# EUROPÄISCHE PATENTSCHRIFT

(11) **EP 2 946 750 B1**
(45) Veröffentlichungstag und Bekanntmachung des Hinweises auf die Patenterteilung: **12.07.2017**
(21) Anmeldenummer: 15168104.6
(22) Anmeldetag: 19.05.2015
(51) Int. Cl.: A61F 2/07

(54) **STENT-GRAFT UND EINFÜHRVORRICHTUNG**
STENT-GRAFT AND DELIVERY SYSTEM
ENDOGRÈFFE ET SYSTÈME D'INSERTION

(30) Priorität: 20.05.2014 DE 102014107113
(43) Veröffentlichungstag der Anmeldung: 25.11.2015
(73) Patentinhaber: Acandis GmbH & Co. KG, 76327 Pfinztal (DE)
(72) Erfinder: Cattaneo, Giorgio, 76199 Karlsruhe (DE); Mailänder, Werner, 75331 Engelsbrand Grunbach (DE); Ding, Andreas, 76135 Karlsruhe (DE)
(74) Vertreter: Kilchert, Jochen

(56) Entgegenhaltungen:
- EP-A2- 2 499 997
- DE-T2- 60 128 588
- DE-U1-202008 014 828
- US-A- 5 282 846
- US-A1- 2006 009 835
- US-B1- 6 287 335

## Beschreibung

Die Erfindung bezieht sich auf eine medizinische Vorrichtung, insbesondere Stent, mit einem komprimierbaren und expandierbaren Geflecht. Eine derartige Vorrichtung mit den Merkmalen des Oberbegriffs des Anspruchs 1 ist beispielsweise aus WO 99/21506 bekannt.

Bei der bekannten medizinischen Vorrichtung handelt es sich um einen Stent Graft, der aus einem Textilmaterial hergestellt ist. Der Stent umfasst ein Geflecht aus unterschiedlichen Filamenten und Drähten. Die Drähte dienen dazu, das Geflecht zu verstärken, das im Übrigen aus Garnsträngen gebildet ist. Bei den Garnsträngen handelt es sich um Fasern, die aus mehreren Einzelfilamenten gebildet sind. Die Garnstränge sind miteinander verwoben, wobei die Verstärkungsdrähte in das Textilmaterial eingeflochten sind. Der bekannte Stent wird als Stentgraft eingesetzt, da das Textilmaterial eine dichte Stentwandung ausbildet. Die Verstärkungsdrähte dienen dazu, dem Stent die erforderliche Radialkraft bei gleichzeitig guter Elastizität sowohl in radialer als auch in Längsrichtung zu verleihen.

Ein weiterer Stent mit unterschiedlichen Filamenten ist aus DE 10 2012 016 301 B3 bekannt. Bei der Herstellung dieses Stents werden Filamente aus dem Geflecht entfernt und durch Filamente aus anderen Materialien ersetzt. Dies gibt die Möglichkeit, die Filamente separat voneinander einer Wärmebehandlung zu unterziehen, wodurch unterschiedliche Eigenschaften der Filamente gezielt eingestellt werden können. Ein Graftmaterial aus Einzelfasern ist auch aus der DE202008014828 U1 bekannt. Der Erfindung liegt die Aufgabe zugrunde, die Porosität der medizinischen Vorrichtung der eingangs genannten Art zu verbessern, wobei die Vorrichtung gut komprimierbar sein soll.

Erfindungsgemäß wird diese Aufgabe durch eine medizinische Vorrichtung mit den Merkmalen des Anspruchs 1 gelöst.

Die Erfindung beruht auf dem Gedanken, eine medizinische Vorrichtung, insbesondere einen Stent, mit einem komprimierbaren und expandierbaren Geflecht anzugeben, das wenigstens einen Verstärkungsdraht und wenigstens einen Faserstrang aufweist. Der Faserstrang ist durch mehrere Einzelfilamente gebildet, d.h. der Faserstrang umfasst mehrere Einzelfilamente. Insbesondere besteht der Faserstrang aus mehreren Einzelfilamenten. Die Einzelfilamente des Faserstranges sind derart lose angeordnet, dass der Faserstrang im komprimierten Zustand des Geflechtes komprimiert und im expandierten Zustand des Geflechtes zumindest teilweise expandiert ist.

Im Stand der Technik wird angestrebt, Faserstränge aus mehreren Einzelfilamenten so stark zu verdrillen, dass eine feste und reproduzierbare Anordnung der Einzelfilamente erreicht wird. Der Querschnitt der Faserstränge soll im komprimierten Zustand des Geflechtes dem Querschnitt der Faserstränge im expandierten Zustand des Geflechtes entsprechen.

Die Erfindung geht einen anderen Weg und ordnet die Einzelfilamente des jeweiligen Faserstranges derart lose an, dass der Faserstrang im komprimierten Zustand des Geflechtes komprimiert und im expandierten Zustand des Geflechtes zumindest teilweise expandiert ist. Die feste Anordnung der Einzelfilamente und damit der stabile Querschnitt des Faserstranges werden aufgegeben. Die erfindungsgemäß lose Anordnung der Einzelfilamente bewirkt, dass der Faserstrang einen veränderbaren Querschnitt mit unterschiedlichen Funktionalitäten aufweist. Beim Übergang vom komprimierten in den expandierten Zustand und umgekehrt ändert sich der Querschnitt des Faserstranges. Die lose Anordnung bedeutet nicht, dass die Einzelfilamente vollkommen frei angeordnet sein müssen. Eine geringfügige Verdrillung der Einzelfilamente wird zugelassen, die die Herstellbarkeit und die Anordnung in komprimierten Zustand erleichtert.

Die Einzelfilamente werden beim Komprimieren des Geflechts derart zusammengedrückt, dass diese aneinander anliegen und einen kompakten, d.h. komprimierten Faserstrang bilden (1. Zustand). Damit wird beim Crimpen der Vorrichtung in einen Katheter, insbesondere Mikrokatheter der Platzbedarf der Faserstränge im Geflecht verringert, so dass ein kleiner Crimpdurchmesser erzielt werden kann.

Der durch die lose Anordnung der Einzelfilamente erreichte variable Querschnitt des Faserstranges bewirkt beim Expandieren des Geflechtes, dass der Faserstrang expandiert (2. Zustand). Die Expansion des Faserstranges beim Übergang vom komprimierten in den expandierten Zustand des Geflechts erfolgt in einer Richtung quer zur Längserstreckung des Faserstranges. Der Faserstrang wird verbreitert. Die Einzelfilamente des jeweiligen Faserstranges verlassen ihre Lage im kompakten Verbund und ordnen sich zumindest teilweise mit Abstand voneinander und/oder in einer anderen Ebene an. Die Einzelfilamente können beim Expandieren des Geflechts zumindest teilweise vereinzelt werden.

Durch die Expansion des Faserstranges wird erreicht, dass seine Einzelfilamente sich jeweils - zumindest teilweise - in der Ebene des Geflechtes ausbreiten bzw. verteilt anordnen. Aufgrund der losen Anordnung der Einzelfilamente im Faserstrang wird die Ausbreitung der Einzelfilamente beim Expandieren automatisch erreicht, da die Komprimierkraft, die durch das Geflecht auf die Faserstränge bzw. den Faserstrang ausgeübt wird, entfällt.

Unter der Expansion des Faserstranges wird allgemein eine Lageänderung seiner Einzelfilamente beim Übergang des Geflechts vom komprimierten in den expandierten Zustand verstanden, die dazu führt, dass die Kontur des Faserstranges in einer Richtung quer zur Längserstreckung des Faserstranges derart verändert wird, dass Geflechtfreiräume überdeckt werden.

Der expandierte Zustand des Geflechtes kann der Zustand ohne Einwirkung von äußeren Kräften (Ruhezustand) oder der implantierte Zustand im Gefäß sein. Im Ruhezustand ist das Geflecht vollexpandiert. Im implantierten Zustand ist das Geflecht teilexpandiert, da die Vorrichtung, insbesondere der Stent, im Ruhezustand überdimensioniert ist, um im implantierten Zustand die erforderliche radiale Haltekraft auf die Gefäßwand aufzubringen. Im teilexpandierten Zustand ist der Durchmesser ca. 0,5 - 1 mm kleiner als der Ruhezustand. In diesem Zustand ist die expandierte, flache Anordnung der Einzelfilamente besonders ausgeprägt.

Im vollexpandierten Zustand können die Einzelfilamenten wieder etwas zusammenrücken, wenn der Flechtwinkel sehr groß, bspw. größer als 75° oder 80° ist. Die Zelle bzw. Masche wird in diesem Fall wieder etwas kleiner, wobei sie nicht wie im komprimierten Zustand in der Länge gestreckt sondern in Umfangsrichtung gestreckt ist.

Die Ausbreitung der Einzelfilamente in der Geflechtebene führt dazu, dass die Porosität des Geflechts verringert wird.

Die Erfindung vereint daher gute Crimpeigenschaften (1. komprimierter Zustand) mit einer signifikant verbesserten Porosität des Geflechtes (2. expandierter Zustand). Mit anderen Worten wird erfindungsgemäß erreicht, dass das Geflecht im expandierten Zustand relativ dicht ist, ohne dass dabei die Komprimierbarkeit der Vorrichtung leidet, weil sich die Einzelfilamente im jeweiligen Zustand optimal anordnen.

Die erfindungsgemäße Vorrichtung eignet sich daher beispielsweise gut für den Einsatz als Flowdiverter, insbesondere zur Behandlung von Aneurysmen. Die Erfindung ist nicht auf Flowdiverter eingeschränkt, sondern kann allgemein für Stentgrafts oder andere medizinische Vorrichtungen eingesetzt werden, bei denen ein Geflecht komprimiert und expandiert wird und eine niedrige Porosität eingestellt werden soll.

Mögliche Einsatzbereiche für das Geflecht ist die Behandlung im intrakraniellen Bereich oder der Halsschlagader. Die Erfindung fördert die Miniaturisierbarkeit der Vorrichtung, so dass diese in besonders kleinen intrakraniellen Gefäßen einsetzbar ist. Aufgrund der niedrigen Porosität des Geflechtes eignet sich dieses besonders gut zur Behandlung von Aneurysmen oder allgemein als Occlusionsdevice zum Verschluss von Hohlräumen. Die Erfindung ist als Stenose-Stent, z.B. im Halsschlagader-Bereich, im intrakraniellen Bereich oder in coronaren oder peripheren Bereichen geeignet. Das dichte Fasergeflecht im expandierten Zustand funktioniert als Filter gegen die Ablösung von Thrombenteilen. Außerdem ermöglicht die hohe Porosität bzw. die Engmaschigkeit des Geflechts vorteilhaft eine gute Zellmigration und fördert insofern die Bildung einer erneuten Endothelschicht.

Die Erfindung ist nicht auf Stents mit röhrchenförmigen Geometrien eingeschränkt, sondern ist für permanente oder temporäre Implantate mit verschiedenen Geometrien geeignet. Beispielsweise kann die erfindungsgemäße Vorrichtung in der Form einer Kugel oder eines anderen Verschlussdevices ausgebildet sein, das beispielsweise zur Behandlung großer Aneurysmen oder anderer Hohlräume, z.B. Vorhofohr geeignet ist. Die Erfindung kann auch zur Behandlung in der Aorta, insbesondere von Aorta-Aneurysmen eingesetzt werden.

Generell ist die Erfindung für die intravaskuläre Behandlung geeignet.

Bei den Einzelfilamenten handelt es sich um Einzelfäden mit einer Länge, die sehr viel größer ist, als deren Durchmesser. Die Länge der Einzelfilamente richtet sich nach den Dimensionen der medizinischen Vorrichtung. Bei der Bestimmung der Länge der Einzelfilamente greift der Fachmann auf an sich bekannte Werte zurück. Der Durchmesser der Einzelfilamente kann beispielsweise einen runden, insbesondere kreisrunden Querschnitt aufweisen. Andere Querschnitte sind möglich.

Im Extremfall kann ein Faserstrang aus zwei Einzelfilamenten gebildet sein. Mehr als zwei Einzelfilamente sind sinnvoll, um die Porosität wirksam zu verringern.

Weitere Vorteile der Erfindung ergeben sich aus den nachfolgenden Ausführungsformen.

So kann das Geflecht wenigstens einen Verstärkungsdraht aufweisen, der zur Bildung von Maschen geflochten ist. Das Geflecht kann wenigstens zwei Verstärkungsdrähte aufweisen, die zur Bildung von Maschen geflochten sind. Die Maschen des Verstärkungsdrahtes bzw. der Verstärkungsdrähte bilden eine Grundstruktur des Geflechtes, die die mechanischen Eigenschaften der Vorrichtung weitgehend bestimmt und den oder die Faserstränge abstützt. Der Verstärkungsdraht bildet damit die Grundeinheit des Geflechts, d.h. die einzelnen Maschen bzw. Zellen, die für eine gute Compliance des Geflechts sorgen.

Das Geflecht kann einlagig oder mehrlagig aufgebaut sein. Das einlagige Geflecht besteht aus den Verstärkungsdrähten und den Fasersträngen, die zumindest teilweise miteinander verflochten sind. Bei dem mehrlagigen Geflecht bilden die Verstärkungsdrähte und die Faserstränge jeweils gesonderte Geflechtlagen, die aufeinander angeordnet sind und zusammen das Geflecht bilden. Es kann auch sein, dass eine Lage die Verstärkungsdrähte und Faserstränge, und die andere Lage nur Faserstränge oder nur Verstärkungsdrähte umfasst

Bei einer bevorzugten Ausführung sind die Einzelfilamente des Faserstranges in wenigstens einer Masche im expandierten Zustand des Geflechts verteilt angeordnet. Die freie Fläche der Maschen, die durch die Verstärkungsdrähte bzw. dem einzelnen Verstärkungsdraht begrenzt ist, wird durch die Einzelfilamente des durchmesservariablen Faserstranges beim Expandieren des Geflechts verringert. Dazu sind die Einzelfilamente des Faserstranges im Bereich der Masche zumindest teilweise verteilt angeordnet. Die Einzelfilamente überdecken damit die Durchtrittsöffnung der Masche bzw. Zelle teilweise. Im Unterschied zu einem Faserstrang mit konstantem Querschnitt wird die Porosität des Geflechts bzw. der einzelnen Maschen des Geflechts verringert. Die Einzelfilamente können homogen in der Masche verteilt angeordnet sein.

Eine weitere Verringerung der Porosität wird dadurch erreicht, dass bei einer besonders bevorzugten Ausführungsform einer Masche mehrere Faserstränge zugeordnet sind, deren Einzelfilamente in der Masche im expandierten Zustand des Geflechts verteilt angeordnet sind. Bei dieser Ausführungsform wird erreicht, dass eine besonders große Anzahl von Einzelfilamenten pro Masche über deren Fläche verteilt angeordnet sind. Der Überdeckungsgrad der Masche wird damit erhöht.

Generell können sowohl alle Maschen des Geflechts durch expandierbare Faserstränge bzw. einen expandierbaren Faserstrang im expandierten Zustand überdeckt sein. Alternativ können auch einzelne Bereiche des Geflechtes durch Faserstränge überdeckt werden, so dass die Porosität des Geflechts lokal einstellbar ist.

Bei einer weiteren bevorzugten Ausführungsform ist der Faserstrang im expandierten Zustand breiter als im komprimierten Zustand. Die Verbreiterung des Faserstranges wird durch die Lageänderung, bspw. durch eine Vereinzelung, der losen angeordneten Einzelfilamente erreicht, die sich in Querrichtung, d.h. senkrecht zur Längserstreckung des Faserstranges beim Expandieren voneinander entfernen. Dabei muss nicht zwingend ein Abstand zwischen den Einzelfilamenten vorliegen. Es genügt, wenn sich die Lage der Einzelfilamente des jeweiligen Faserstranges beim Übergang vom komprimierten Geflechtzustand in den expandierten Geflechtzustand so ändert, dass die Überdeckung der Geflechtebene durch die Einzelfilamente erhöht wird. Mit anderen Worten steigt die Anzahl der Einzelfilamente, die in Breitenrichtung bzw. allgemein in einer Richtung quer zur Längserstreckung der Faserstränge bzw. des jeweiligen Faserstranges angeordnet sind. Dementsprechend nimmt die Anzahl der Einzelfilamente, die in Höhenrichtung bzw. bei röhrchenförmigen Vorrichtung in radialer Richtung des jeweiligen Faserstranges angeordnet sind, ab.

Eine besonders gute Abdeckung des Geflechts wird erreicht, wenn die Einzelfilamente zumindest teilweise planar angeordnet sind. Die planare Anordnung bezieht sich auf die Geflechtebene. Mit anderen Worten sind die Einzelfilamente im expandierten Zustand des Faserstranges in ein und derselben Ebene nebeneinander, d.h. planar angeordnet. Es kann ausreichend sein, wenn zumindest ein Teil der Einzelfilamente planar angeordnet ist, beispielsweise zwei oder mehr Einzelfilamente. Die übrigen Einzelfilamente können in Höhenrichtung, d.h. im Wesentlichen quer zur Geflechtebene angeordnet sein, wobei auch bei diesen Einzelfilamenten eine Lageänderung gegenüber dem komprimierten Zustand des Faserstranges eintritt. Die Einzelfilamente können sich in Breitenrichtung des Faserstranges, d.h. quer zur Längserstreckung bewegen, so dass der Faserstrang insgesamt im Vergleich zum komprimierten Zustand flacher wird.

Damit wird zumindest im Bereich des jeweiligen Faserstranges bzw. des wenigstens einen Faserstranges die Wandstärke des Geflechts verringert.

Bei einer weiteren bevorzugten Ausführungsform beträgt das Verhältnis von h/b expandiert zu h/b komprimiert von 0,01 bis 0,8, wobei h die Höhe des Faserstranges und b die Breite des Faserstranges bedeuten. Die Höhe h wird im Wesentlichen senkrecht zur Geflechtebene, bei gewölbten Geflechten, wie Stents oder dgl., in radialer Richtung bestimmt. Die Breite b wird in der Geflechtebene, bei gewölbten Geflechten, wie Stents oder dgl., in Umfangsrichtung bestimmt.

Die Obergrenze für das Verhältnis (h/b)_{expandiert} / (h/b)ₖₒₘₚᵣᵢₘᵢₑᵣₜ beträgt 0,8, insbesondere 0,6, insbesondere 0,4, insbesondere 0,2, insbesondere 0,1. Mit abnehmender Obergrenze wird der geometrische Unterschied zwischen komprimiertem und expandiertem Zustand des Faserstranges stärker ausgeprägt. Die Überdeckung der Masche bzw. der Maschen im expandierten Zustand nimmt zu.

Die Untergrenze für das Verhältnis (h/b)_{expandiert} / (h/b)ₖₒₘₚᵣᵢₘᵢₑᵣₜ beträgt 0,01, insbesondere 0,05.

Das obige Verhältnis (h/b)_{expandiert} / (h/b)ₖₒₘₚᵣᵢₘᵢₑᵣₜ gilt zumindest innerhalb der Zelle bzw. Masche, also zwischen zwei Verstärkungsdrähten. Es kann entlang des ganzen Geflechtes gelten. Es kann auch im Bereich der Überkreuzungen gelten.

Bei einer weiteren Ausführungsform kann der Faserstrang von 5 bis 50 Einzelfilamente, insbesondere von 20 bis 30 Einzelfilamente aufweisen. Bei mehreren Fasersträngen kann jeder einzelne Faserstrang von 5 bis 50 Einzelfilamente, insbesondere von 20 bis 30 Einzelfilamente aufweisen. Das Einzelfilament kann einen runden, insbesondere kreisrunden Querschnitt mit einem Durchmesser von 5 bis 30 µm, insbesondere von 10 bis 20 µm aufweisen.

Die lose Bündelung kann vorzugsweise durch den Verdrillungsgrad des jeweiligen Faserstrangs eingestellt werden. Der Verdrillungsgrad wird durch die Anzahl der Umdrehungen der Einzelfilamente pro Meter gemessen. Es hat sich gezeigt, dass in Abhängigkeit von der Anzahl der Einzelfilamente pro Faserstrang unterschiedliche Bereiche des Verdrillungsgrades zu einer ausreichend losen Anordnung der Einzelfilamente führen. Der Verdrillungsgrad in Umdrehungen pro Meter eines Faserstranges von 50 bis 800, insbesondere von 50 bis 700, insbesondere von 50 bis 600, insbesondere von betragen.

Allgemein ist es nicht erforderlich, dass die Verdrillung der Einzeldrähte komplett entfällt. Es genügt, wenn der Verdrillungsgrad der Einzelfilamente so verringert wird, dass eine Expansion des jeweiligen Faserstranges beim Übergang des Geflechts vom komprimierten auf den expandierten Zustand möglich ist.

Folgende Beispiele des Verdrillungsgrades bei unterschiedlich starken Fasersträngen werden offenbart:
- Verdrillungsgrad (in Umdrehungen pro Meter), z.B. bei 10 Einzelfilamenten:
   ∘ kleiner als 800, insbesondere 700, insbesondere 600, insbesondere 500
   ∘ größer als 300
- Verdrillungsgrad (in Umdrehungen pro Meter), z.B. bei 25 Einzelfilamenten:
   ∘ kleiner als 500, insbesondere 400, insbesondere 300, insbesondere 200
   ∘ größer als 50
- Verdrillungsgrad (in Umdrehungen pro Meter), z.B. bei 50 Einzelfilamenten:
   ∘ kleiner als 300, insbesondere 200, insbesondere 150
   ∘ größer als 50

Allgemein erfolgt die Verdrillung entlang der Längsachse des Faserstranges bzw. bei einer röhrchenförmigen Vorrichtung in Längsrichtung der Vorrichtung.

Andere Möglichkeiten der losen Bündelung der Einzelfilamente zu einem Faserstrang als durch Verdrillung sind denkbar.

Die Anzahl der Verstärkungsdrähte kann von 6 bis 32 betragen. Vorzugweise kann die Anzahl der Verstärkungsdrähte von 8 bis 24, insbesondere von 12 bis 20, betragen.

Es ist möglich, dass die Verstärkungsdrähte an einem Ende des Geflechts umgelenkt sind. In diesem Fall kann die Anzahl der Verstärkungsdrähte von 3 bis 16, insbesondere von 4 bis 12, insbesondere von 6 bis 10, betragen. Da die Verstärkungsdrähte an einem Ende des Geflechts umgelenkt sind, entspricht die Anzahl der Verstärkungsdrähte, die bei einer Querschnittsbetrachtung des Geflechtes zu sehen sind, jedoch der im vorangegangenen Absatz erwähnten Anzahl von Verstärkungsdrähten. Sofern die Verstärkungsdrähte umgelenkt sind gilt somit, dass die reelle Anzahl der Drähte, die im Geflecht eingeflochten sind, reduziert ist. Bei einer einzigen Umlenkung ist die Drahtanzahl beispielsweise halbiert. Bei zwei Umlenkungen pro Draht enspricht die Anzahl der vorhandenen Drähte einem Drittel der Anzahl der Drähte eines Geflechts, bei dem die Drähte nicht umgelenkt sind. Bei einem Geflecht, das aus einem einzigen Verstärkungsdraht gebildet ist, entspricht die Gesamtzahl der Umlenkungen an beiden Enden des Geflechts der Anzahl der Verstärkungsdrähte, die auf einem Querschnitt durch das Geflecht zu erkennen sind.

Im Extremfall kann auch ein einziger Verstärkungsdraht vorgesehen sein, der derart an den Geflechtenden umgelenkt ist, dass die Bildung eines Maschengeflechts bzw. Gittergeflechts ermöglicht wird. In diesem Fall kann der Verstärkungsdraht an den Geflechtenden derart oft umgelenkt sein, dass ein, im Querschnitt zu betrachtendes Geflecht gebildet ist, das die in einem vorherigen Absatz genannte Anzahl der Verstärkungsdrähte, nämlich von 6 bis 32, insbesondere von 8 bis 24, insbesondere von 12 bis 20, aufweist. Bei einer derartigen Ausführungsform der Erfindung können freie Enden des Verstärkungsdrahtes, insbesondere mit Hilfe einer Crimphülse, miteinander verbunden sein.

Die Erklärungen hinsichtlich umgelenkter Verstärkungsdrähte und deren Anzahl gelten auch für die Anzahl von Fasersträngen, die in der nachfolgenden Beschreibung erläutert sind. Als Anzahl ist Folgenden die Anzahl von Verstärkungsdrähten und/oder von Fasersträngen zu verstehen, die auf einem Querschnitt der medizinischen Vorrichtung zu sehen sind, unabhängig davon, ob und wie häufig die Verstärkungsdrähte und/oder Faserstränge an einem Ende des Geflechts umgelenkt sind.

Die Anzahl der Faserstränge kann von 12 bis 128 betragen. Mit zunehmender Anzahl der Verstärkungsdrähte steigt die Radialkraft im Fall einer rohrförmigen medizinischen Vorrichtung, insbesondere eines Stents. Mit steigender Anzahl der Fasern mit lose gebündelten Einzelfilamenten wird der Überdeckungsgrad, der mit dem Geflecht erreicht wird, erhöht. Es ist möglich, dass mindestens ein Faserstrang an einem Ende des Geflechtes mindestens einmal umgelenkt ist. Die effektive Anzahl der Faserstränge kann in einer derartigen Ausführungsform reduziert werden. Der Faserstrang kann auch mehrmals an einem Ende bzw. an beiden Enden des Geflechts umgelenkt sein. In diesem Zusammenhang ist es möglich, dass ein Faserstrang oder zwei Faserstränge oder drei Faserstränge oder vier Faserstränge durch entsprechende Umlenkung(en) am Ende oder an beiden Enden des Geflechtes bei Betrachtung des Geflechtes im Querschnitt, ein Geflecht mit 12 bis 128 Fasersträngen bildet bzw. bilden.

In einer möglichen Ausführungsform der Erfindung können in einem Geflecht mit zwei Fasersträngen diese beiden Faserstränge durch die gleiche Zelle des Geflechts, die aus Verstärkungsdrähten gebildet ist, verlaufen. Mit anderen Worten können zwischen zwei Verstärkungsdrähten zwei Faserstränge angeordnet sein. Für jeden Verstärkungsdraht können zwei Faserstränge eingeflochten sein. Dies bedeutet, dass ein Verstärkungsdraht und zwei Faserstränge auf der Mantelfläche des Geflechts parallel zueinander verlaufen und an den Enden des Geflechts an unterschiedlichen Stellen umgelenkt sind. Jeweils die beiden Enden eines Verstärkungsdrahten oder eines Faserstranges können miteinander verbunden sein. Die Enden der Faserstränge können stirnflächig oder überlappend miteinander verbunden, insbesondere verschweißt, sein. Bei alleiniger Betrachtung der Verstärkungsdrähte oder der Faserstränge, bilden die Verstärkungsdrähte oder die Faserstränge ein vollständiges Geflecht und/oder ein Netz und/oder eine Gitterstruktur aus sehr breiten Zellen. Das Verflechten der fiktiven Einzelgeflechte bildet ein gesamtes, feinmaschiges Geflecht und bewirkt eine große Stabilität des Geflechts.

Das Verhältnis der Anzahl von Verstärkungsdraht zu Faserstrang kann von 1:12 bis 1:1, insbesondere von 1:6 bis 1:2, insbesondere von 1:4 bis 1:3, betragen. Auch hier gilt, dass mit fallendem Verhältnis die Porosität des Geflechtes im expandierten Zustand abnimmt. Umgekehrt steigt mit einem großen Verhältnis Anzahl Verstärkungsdraht/Anzahl Faserstrang die erzielbare Radialkraft des Geflechts im Fall einer rohrförmigen Vorrichtung bzw. allgemein im Fall einer Vorrichtung mit gewölbtem bzw. gekrümmtem Geflecht. Das angegebene Verhältnis der Anzahl von Verstärkungsdraht zu Faserstrang gilt auch in Zusammenhang mit den Ausführungsbeispielen, die umgelenkte Verstärkungsdrähte und/oder umgelenkte Faserstränge aufweisen. Das Verhältnis betrifft dann die Anzahl von Verstärkungsdraht zu Faserstrang, die im Querschnitt durch das Geflecht zu erkennen sind.

Durch eine Zelle des Geflechtes können zwischen 2 und 6, insbesondere zwischen 3 und 4, Faserstränge verlaufen. Es ist auch möglich, dass lediglich ein Faserstrang durch eine Zelle des Geflechtes verläuft.

Der Flechtwinkel kann im expandierten Zustand von 45° bis 80° betragen. Unter einem Flechtwinkel wird im Rahmen der Anmeldung der Winkel verstanden, der zwischen einer imaginären Linie, die in Längsrichtung des Geflechts, insbesondere in Längsrichtung des röhrchenförmigen Geflechts und einem Verstärkungsdraht eingeschlossen ist. Mit anderen Worten handelt es sich bei dem Flechtwinkel um die Hälfte des Winkels zwischen zwei sich kreuzenden Verstärkungsdrähten bzw. Maschendrähten.

Der Durchmesser des Verstärkungsdrahtes kann von 25 bis 150 µm, insbesondere zwischen 35 und 100 µm, insbesondere zwischen 50 und 80 µm betragen. Dasselbe gilt für den Durchmesser der übrigen Verstärkungsdrähte.

Es hat sich als besonders vorteilhaft erwiesen, wenn der wenigstens eine Verstärkungsdraht und der wenigstens eine Faserstrang in der Flechtart eins über zwei angeordnet sind. Dadurch wird erreicht, dass sich die Einzelfilamente beim Expandieren des Geflechts gut verteilen. Außerdem wird die Komprimierbarkeit des Geflechts begünstigt. Es sind auch alternative Flechtarten möglich. Beispielsweise kann der wenigstens eine Verstärkungsdraht und der wenigstens eine Faserstrang in der Flechtart eins über eins angeordnet sein.

Bei einer besonders bevorzugten Ausführungsform ist wenigstens ein Faserstrang parallel zu dem wenigsten einen Verstärkungsdraht angeordnet. Es können alle Faserstränge parallel zu dem Verstärkungsdraht bzw. parallel zu den Verstärkungsdrähten angeordnet sein. Die Faserstränge verlaufen entsprechend den parallel verlaufenden Verstärkungsdrähten. Sie folgen dem gleichen Verlauf (unten, oben ...) bei kreuzenden Verstärkungsdrähten. Sie sind mit anderen kreuzenden Verstärkungsdrähten verflochten. Dadurch werden die Bewegungsfreiheit des Geflechtes und damit die Flexibilität der Verstärkungsdrähte erhöht. Die Belastung der Maschen wird verringert.

Es ist möglich, dass ein Faserstrang den gleichen Verlauf wie wenigstens ein Verstärkungsdraht aufweist, jedoch in den Überkreuzungspunkten anders, insbesondere gegensinnig, zum Verstärkungsdraht verläuft. Beispielsweise können ein Verstärkungsdraht und ein Faserstrang zwischen zwei Überkreuzungspunkten im Geflecht nebeneinander, insbesondere parallel zueinander, verlaufen. In einem Überkreuzungspunkt zu einen weiteren Draht oder Strang kann beispielsweise der Verstärkungsdraht oberhalb und der Faserstrang unterhalb des weiteren Drahtes oder Stranges verlaufen. Es ist auch möglich, dass der Faserstrang oberhalb und der Verstärkungsdraht unterhalb des weiteren Drahtes oder Stranges verläuft. Ein Verstärkungsdraht und ein Faserstrang sind dann als parallel zueinander verlaufend zu verstehen, wenn der Verstärkungsdraht und der Faserstrang über eine bestimmte Strecke hinweg, insbesondere mindestens über einen Überkreuzungspunkt, insbesondere über mindestens zwei Überkreuzungspunkte, hinweg, den gleichen Verlauf aufweisen und in diesen Überkreuzungspunkten beide oberhalb oder beide unterhalb des gegengerichteten Drahtes oder Stranges verlaufen.

Die Parallelanordnung kann auf den distalen und/oder proximalen Randbereich des Geflechtes, insbesondere der röhrchenförmigen Vorrichtung bzw. des Stents beschränkt sein. Der so ausgebildete Randbereich kann von 5% bis 20% der Gesamtlänge der Vorrichtung bzw. des Stents betragen. Andere Stentbereiche können mit Parallelanordnung ausgebildet sein.

Zusätzlich oder alternativ kann wenigstens ein weiterer Faserstrang zumindest mit dem Verstärkungsdraht verflochten sein. Der Faserstrang folgt nicht dem Verlauf des parallel laufenden Verstärkungsdrahts, sondern verhält sich wie ein weiter Draht, der im Geflecht angeordnet sind. Diese Anordnung bietet sich für den mittleren Geflechtteil, insbesondere für den mittleren Abschnitt der röhrchenförmigen Vorrichtung bzw. des Stents zwischen den Randbereichen, an, weil dadurch die Porosität lokal verringert wird. Im Allgemeinen verlaufen in der bevorzugten Ausführung normal verflochtene Drähte über die gesamte Länge. Optional werden parallel verlaufende Drähte integriert.

Allgemein kann die Anordnung der Faserstränge zumindest teilweise der Geflechtstruktur, die durch den Verstärkungsdraht bzw. die Verstärkungsdrähte bestimmt ist, angepasst sein. Dabei können alle Faserstränge jeweils parallel zu einem Verstärkungsdraht verlaufen oder geflochten angeordnet sein. Der Verlauf der Faserstränge entspricht dem Verlauf der Verstärkungsdrähte im Geflecht, wodurch eine besonders gute Komprimierbarkeit und homogene Verteilung der Einzelfilamente erreicht wird.

Die Geflechteigenschaften können gezielt, insbesondere lokal gezielt, beeinflusst werden, wenn ein Faserstrang unterschiedliche Einzelfilamente, bspw. aus verschiedenen Materialien oder Geometrien, aufweist und/oder mehrere Faserstränge unterschiedlich viele Einzelfilamente aufweisen.

Die Erfindung umfasst ein System aufweisend eine erfindungsgemäße medizinische Vorrichtung, insbesondere Stent, und ein Zufuhrsystem, insbesondere eine Schleuse und einen Führungsdraht, wobei der Außendurchmesser der Vorrichtung, insbesondere des Stents, im expandierten Ruhezustand höchstens 6 mm und der Innendurchmesser des Zufuhrsystems höchstens 0,7 mm betragen.

Die Erfindung wird nachfolgend mit weiteren Einzelheiten mit Bezug auf die beigefügten schematischen Darstellungen näher beschrieben.

In diesen zeigen:
- Fig. 1:: einen Geflechtausschnitt im Bereich einer Masche im komprimierten Zustand (Stand der Technik);
- Fig. 2:: den Geflechtausschnitt gemäß Fig. 1 im expandierten Zustand (Stand der Technik);
- Fig. 3:: ein größerer Geflechtausschnitt gemäß Fig. 1 (Stand der Technik);
- Fig. 4:: einen Geflechtausschnitt eines Ausführungsbeispiels nach der Erfindung im expandierten Zustand;
- Fig. 5:: einen Querschnitt eines Faserstranges im komprimierten Zustand nach einem erfindungsgemäßen Ausführungsbeispiel;
- Fig. 6:: einen Querschnitt durch einen Faserstrang im expandierten Zustand nach einem erfindungsgemäßen Ausführungsbeispiel;
- Fig. 7:: einen Querschnitt eines Faserstranges im expandierten Zustand nach einem weiteren erfindungsgemäßen Ausführungsbeispiel;
- Fig. 8:: einen Querschnitt durch eine Masche im komprimierten Zustand;
- Fig. 9:: ein Beispiel zur Bestimmung der Filamentdichte und
- Fig. 10:: einen Querschnitt durch eine Masche im expandierten Zustand.

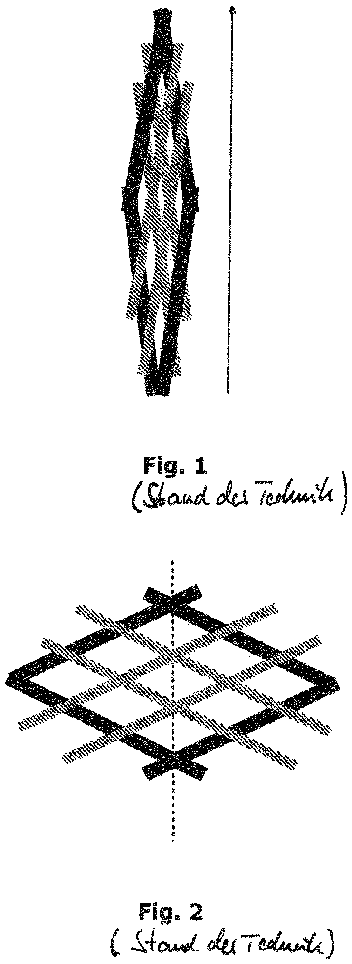

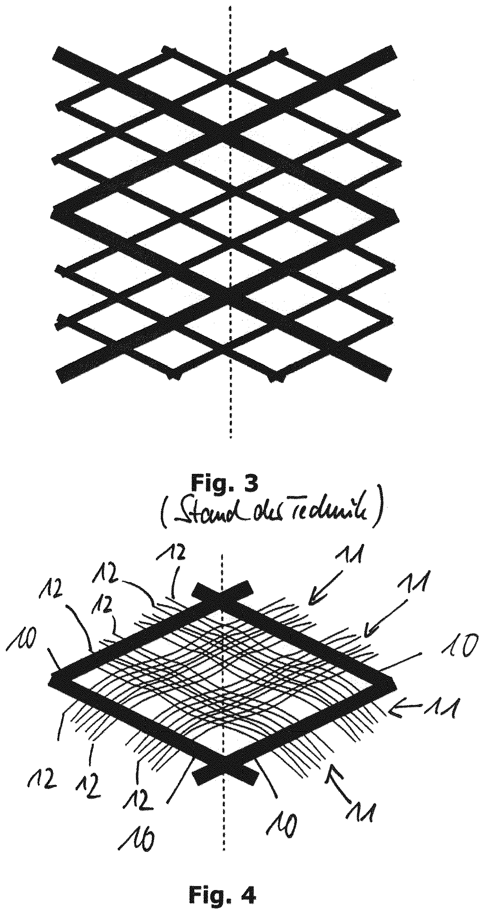

Die Figuren 1-3 zeigen Geflechtausschnitte einer Vorrichtung nach dem Stand der Technik, beispielsweise nach WO 99/021506 A2. Bei diesem Geflecht handelt es sich um einen Stentgraft, dessen Geflecht aus Verstärkungsdrähten und Fasersträngen zusammengesetzt ist. Die Faserstränge sind, wie in den Figuren 1-3 zu sehen, kompakte Stränge mit einem Durchmesser, der im expandierten und komprimierten Zustand des Geflechts konstant ist. Bei Figuren 1 und 2 sind die Verstärkungsdrähte schwarz und die Faserstränge gestrichelt eingezeichnet. Die Stentachse ist durch den senkrechten Pfeil beschrieben. Wie in den Figuren 2 und 3 gut zu erkennen, ändert sich der Durchmesser der Faserstränge beim Übergang vom komprimierten in den expandierten Zustand des Geflechts nicht. Bei der Ausbreitung der Zelle durch die Expansion beabstanden sich die Faserstränge als solche voneinander. Mit anderen Worten ordnen sich diese mit einem relativ großen Abstand voneinander in der Zelle an, die aus den Verstärkungsdrähten gebildet ist. Damit wird eine relativ hohe Porosität erzeugt. Die Anzahl der Fasern bzw. der Drähte beschränkt in der Regel die Zuführbarkeit in Mikrokathetern. Anders formuliert, ist bei einem erforderlichen maximalen Durchmesser des Katheterlumens nur eine maximale Anzahl von Drähten möglich. Wenn der Stent expandiert, bestimmt die Anzahl der Drähte/Filamenten die Dichte des Geflechtes. Die Porengröße und die Porosität können somit einen gewissen Wert nicht unterschreiten.

Im Unterschied zum Stand der Technik sind bei dem erfindungsgemäßem Ausführungsbeispiel gemäß Fig. 4 die Einzelfilamente 12 der Faserstränge 11 lose angeordnet. Dies führt dazu, dass bei der Expansion des Geflechtes die Kraft, die die Einzelfilamente in der gepackten Konfiguration zusammenhält, entfällt. Die Einzelfilamente 11 können sich dadurch homogen oder zumindest teilweise in der Masche bzw. Zelle des Geflechts verteilen. Dadurch wird die Porosität verringert, wie der Vergleich der Figuren 2 und 4 bei entsprechender Anzahl von Fasersträngen (hier: 4 pro Zelle) verdeutlicht. Bei dem vorgestellten Geflecht sind die Einzelfilamente 12 so lose angeordnet, dass diese sich homogen innerhalb einer Zelle oder Masche, die aus Verstärkungsdrähten 10 geformt ist, so anordnen, dass die Porengröße verringert wird.

Wie Fig. 4 zu entnehmen, verteilen sich die Einzelfilamente 12 über die Fläche, die durch die Verstärkungsdrähte 10 aufgespannt ist. Mit anderen Worten verteilen sich die Einzelfilamente über die Masche zwischen den maschenbildenden Verstärkungsdrähten 10 und sind damit in der Zelle bzw. Masche angeordnet. Dazu sind die Einzelfilamenten 12 im Wesentlichen lose d.h. nur minimal gebündelt im Faserstrang 11 angeordnet. Ein Abstand zwischen den Einzelfilamenten 12 entsteht, sobald die Zelle aus Verstärkungsdrähten 10 sich bei der Expansion ausweitet. Dabei sinkt die Kraft, die die Einzelfilamente 12 in gepackter Konfiguration zusammenhält, so dass sich diese homogen innerhalb der Zelle ausbreiten. Der Ausbreitungsgrad hängt davon ab, wie stark der jeweilige Faserstrang verdrillt ist.

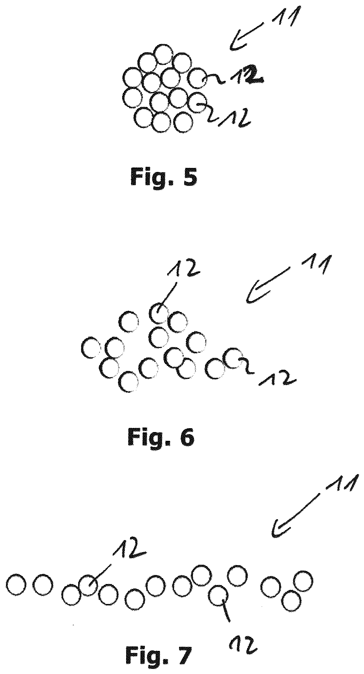

Die Figuren 5, 6 und 7 zeigen, dass sich die Form des Faserstranges beim Übergang vom komprimierten in den expandierten Geflechtzustand ändert. Die Änderung des Querschnitts bzw. allgemein der Form des Faserstranges entspricht einer Expansion in Querrichtung, d.h. senkrecht zur Längserstreckung des Faserstranges 11, wenn die durch das komprimierte Geflecht ausgeübte Haltekraft auf den Faserstrang 11 entfällt. Die Lageänderung der Einzelfilamente 12 innerhalb des Faserstranges 11 führt dazu, dass der Faserstrang die in Fig. 5 gezeigte im Wesentlichen kreisrunde Querschnittsform verliert. Die Einzelfilamente bewegen sich zumindest teilweise seitlich voneinander weg bzw. entfernen sich seitlich voneinander, so dass die Form bzw. der Querschnitt des Faserstranges 11 flacher wird bzw. sich ovalisiert. Mit anderen Worten werden, wie in den Figuren 6 und 7 dargestellt, die Einzelfilamente 12 im expandierten Zustand des Geflechts bzw. somit auch des Faserstranges 11 nicht mehr zusammengehalten, sondern entfernen sich zumindest teilweise seitlich voneinander.

Die Einzelfilamente 12 breiten sich lateral in der Masche aus.

Die Breite des Faserstranges 11 nimmt zu. Die Höhe nimmt entsprechend ab. Insgesamt wird also die Breite des Faserstranges 11 größer als dessen Höhe. Die Dimension Breite" des Faserstranges 11 bezieht sich auf die Anordnung des Faserstranges im Geflecht. Die Breitenrichtung verläuft damit in Geflechtebene. Die Höhenrichtung verläuft senkrecht zur Geflechtebene. Im Fall eines Stents bzw. rohrförmigen Geflechtes verläuft die Breitenrichtung in Umfangsrichtung des Stents und die Höhenrichtung in radialer Richtung des Stents.

Die Figuren 6 und 7 unterscheiden sich im Verdrillungsgrad des jeweiligen Faserstranges 11. Bei dem Faserstrang 11 gemäß Fig. 6 ist der Verdrillungsgrad der Einzelfilamente 12 etwas höher als bei dem Faserstrang 11 gemäß Fig. 7. Dies zeigt sich darin, dass die Ausdehnung des Faserstranges 11 in Breitenrichtung bei dem Beispiel gemäß Fig. 6 weniger stark ausgeprägt ist als bei dem Beispiel gemäß Fig. 7. Mit anderen Worten sind bei dem Beispiel gemäß Fig. 6 mehr Einzelfilamente 12 in Höhenrichtung angeordnet, d.h. übereinander angeordnet als bei dem Beispiel gemäß Fig. 7. Das Beispiel gemäß Fig. 7 zeigt, dass es bei einer vergleichsweise niedrigen Verdrillung möglich ist, die Einzelfilamente 12 nahezu planar, d.h. in der Geflechtebene anzuordnen. Diese Lage der Einzelfilamente 12 richtet sich nach dem Verdrillungsgrad, der Anzahl der Drähte bzw. der Anzahl der Einzelfilamente und der Flechtart, beispielsweise eins über zwei. Es kann sein, dass die Faserstränge 11 in Figuren 6, 7 die selbe Zusammensetzung haben (Anzahl und Durchmesser der Einzelfilamenten und Verdrillungsgrad), das Geflecht allerdings selbst unterschiedliche Eigenschaften (Flechtwinkel, Flechtart,...) aufweist.

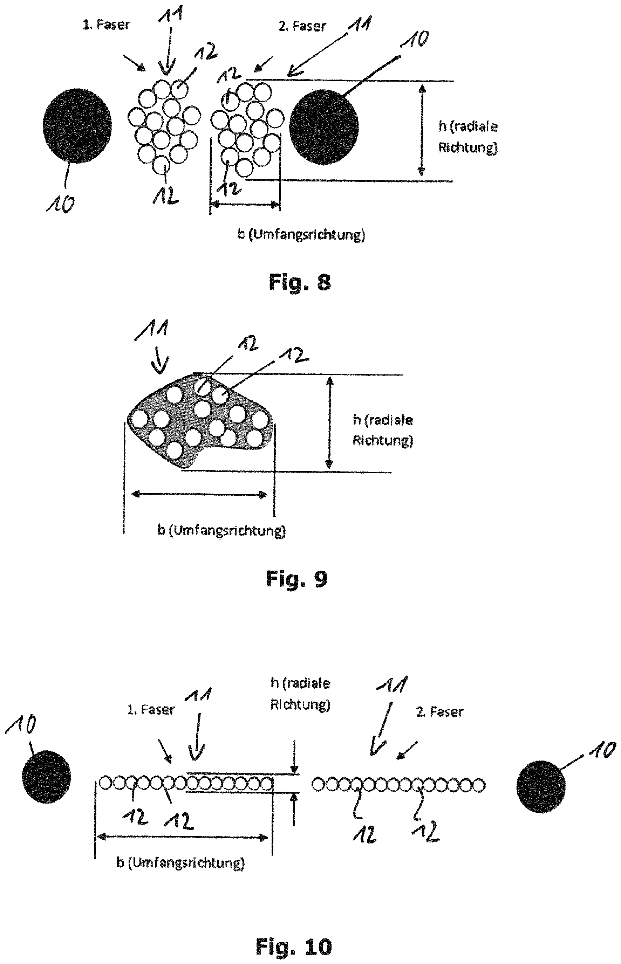

Nachfolgend werden anhand der Figuren 8, 9 die Geometrie der Faserstränge 11 im komprimierten Zustand und die Filamentdichte als Kriterium für die Querschnittsänderung des Faserstranges 11 erläutert.

Die Fasern sind schwach verdrillt, also auch im komprimierten Zustand des Geflechtes. Werte über die Verdrillung, insbesondere die Obergrenze, werden unten angegeben.

Im komprimierten Zustand sind die Einzelfilamente 12 durch die benachbarten Verstärkungsdrähte 10 zusammengedrückt bzw. komprimiert. Sie erreichen daher in der Regel eine Dichte, die wesentlich höher als im expandierten Zustand ist, da die äußeren Kräfte wesentlich geringer sind. Anders als bei einer starken Verdrillung, ordnen sich die Einzelfilamente 12 aber so an, dass kein runder Querschnitt bildet wird. Es ergibt sich eine Konfiguration in komprimiertem Zustand, bei der der Querschnitt der Faser im Wesentlichen oval ist, wie in Fig. 8 zu sehen. Es handelt sich dabei nicht um ein geometrisch exaktes Oval (s. 1. und 2. Faser in Fig. 8). Allgemein ist der Durchmesser des Faserstranges in einer ersten Richtung, bspw. die Höhe h in radialer Richtung, größer als der Durchmesser des Faserstranges in einer zweiten Richtung senkrecht zur ersten Richtung, bspw. die Breite b in Umfangsrichtung. Nach Art des Geflechtes ist das Gegenteil möglich.

Die Richtungsangaben beziehen sich auf die Lage des Faserstranges 11 im Stent bzw. allgemein auf die gekrümmte Geflechtwandung.

Die Obergrenze für das Verhältnis h/b ist 0,9, insbesondere 0,8, insbesondere 0,6, insbesondere 0,4 im komprimierten Zustand des Geflechtes. Die Untergrenze beträgt 0,2. Die ovale Form muss nicht exakt sein. Es geht vielmehr darum, dass die resultierende Form in zwei zueinander senkrechten Richtungen unterschiedliche Dimensionen aufweist.

Es kann auch sein, dass die ovale Form eine andere Ausrichtung als gezeigt aufweist.

Im komprimierten Zustand kann der Strangquerschnitt rund oder nahezu rund sein.

Der Querschnitt, insbesondere die Form und die Dimensionen, kann sich entlang der Faser ändern. Bspw. ist die ovale Form zwischen zwei Drähten wie in Fig. 8 gebildet. Im Bereich der Überkreuzungen, an denen die Fasern oberhalb bzw. unterhalb der Verstärkungsdrähte verlaufen, kann sich die Form ändern. Bspw. wird hier die ovale Form lokal abgeflacht.

Die Breite b ist größer als die Höhe h und der Faserstrang 11 erstreckt sich mehr in Umfangsrichtung als in radialer Richtung. Die Breite der Faser in expandiertem Zustand ist mindestens 0,5 x Anzahl der Einzelfilamente x Durchmesser der Einzelfilamente, mindestens 0,8, insbesondere 1,0, insbesondere 1,2, insbesondere 1,5. In absoluten Werten ist die Untergrenze der Breite in expandiertem Zustand 100 µm, insbesondere 150 µm, insbesondere 200 µm, insbesondere 250 µm, insbesondere 300 µm, insbesondere 350 µm, insbesondere 400 µm, insbesondere 450 µm, insbesondere 500 µm. Obergrenze ist 800 µm, insbesondere 600 µm.

Dies ist ein weiterer Vorteil der losen Bündelung der Einzelfilamente 12, weil der Außendurchmesser der Vorrichtung im komprimierten Zustand im Bereich der Überkreuzungen im Vergleich zu bekannten Geflechten aus formstabilen Filamenten mit konstantem Filamentdurchmesser kleiner ist.

Allgemein sind die Faserstränge 11 forminstabil.

Ein weiteres, mögliches Kriterium für die Beschreibung der Faserstränge 11 ist die Dichte der Einzelfilamente 12 im Faserstrang 11. Dazu wird auf Fig. 9 verwiesen, die zeigt, dass die Dichte die Summe der Querschnittsflächen der Einzelfilamente 12 bezogen auf die Gesamtschnittfläche des Faserstranges 11 darstellt. Die Gesamtschnittfläche ergibt sich aus der Außenkontur des Faserstranges 11, die durch die Anordnung der Einzelfilamente 12 begrenzt ist (Fig. 9). Die Dichte des Faserstranges 11 wird dadurch gemessen, dass das komprimierte Geflecht in ein Harz eingebettet, gehärtet und dann geschnitten wird.

Die Dichte eines aus verdrillten Einzelfilamenten bestehenden Faserstranges 11 beträgt im komprimierten Zustand in der Regel höchstens 78 %, insbesondere höchstens 75%, insbesondere höchstens 70%, insbesondere höchstens 60%. Die Untergrenze ist 40%, insbesondere 50%.

Die Dichte in expandierter Konfiguration kann ebenfalls 78%, insbesondere 75%, insbesondere 70%, insbesondere 60% als Obergrenze betragen und zwar wenn die Einzelfilamente in Reihe ohne Zwischenspalt bzw. Abstand angeordnet sind (Fig. 10). Der Unterschied zwischen dem komprimierten und expandierten Zustand besteht in der Querschnittsgeometrie des Faserstranges 11, insbesondere zwischen den Verstärkungsdrähten 10. Trotzdem resultiert eine höhere Abdeckung der Zelle bzw. Masche als in dem Fall, wenn der Faserstrang verdrillt ist, weil sich die Einzelfilamente 12 alle auf einer Linie bzw. in einer Ebene innerhalb der Zelle anordnen oder zumindest eine flache Kontur annehmen können

Im expandierten Zustand des Geflechtes ändert sich die Anordnung der Einzelfilamente 12. Die ovale Querschnittsformändert sich und das Verhältnis h/b nimmt ab. Im expandierten Zustand des Geflechtes beträgt das Verhältnis h/b wenigstens abschnittsweise entlang des Geflechtes, insbesondere im Bereich zwischen den Verstärkungsdrähten 10 (nicht im Überkreuzungsbereich) höchstens 0,6, insbesondere höchstens 0,4, insbesondere höchstens 0,2. Die Untergrenze beträgt 0,05, insbesondere 0,1.

Die Dichte im expandierten Zustand kann geringer als vorstehend erläutert sein, weil sich die Einzelfilamente 12 in lateraler Richtung voneinander trennen können. In Abhängigkeit von der Flechtart und Flechtkonfiguration kann eine Dichte von höchstens 70%, insbesondere höchstens 60%, insbesondere höchstens 50%" insbesondere höchstens 40% eingestellt werden. Die Untergrenze kann 20%, insbesondere 30%, insbesondere 35% betragen.

Das Verhältnis der Dichte im expandierten und komprimierten Zustand beträgt, höchstens 0,9, insbesondere höchstens 0,8, insbesondere höchstens 0,7, insbesondere höchstens 0,6, insbesondere höchstens 0,5. Im Spezialfall, dass die Einzelfilamente 12 sowohl im komprimierten als auch im expandierten Zustand aneinander anliegen und nur die Querschnittsgeometrie variiert, beträgt das Verhältnis der Dichte 1. Durch die flache Anordnung der Einzelfilamente entstehen kleinere Poren im Geflecht in teilexpandiertem Zustand. Die maximale Porengröße (= Durchmesser des in die Masche eingeschriebenen Kreises) innerhalb einer Zelle beträgt 150 µm, insbesondere 125 µm, insbesondere 100 µm, insbesondere 75 µm, insbesondere 50 µm. Die Untergrenze ist 10 µm. Die Porosität des Geflechtes beträgt maximal 60 %, insbesondere 50 %, insbesondere 40%, insbesondere 30%, insbesondere 20%, insbesondere 15%. Die Untergrenze ist 5%, insbesondere 10%. Dies ist günstig für die Versorgung des Gefäßendotheliums und die Durchblutung von Seitengefäßen.

Im komprimierten Zustand können sich die Einzelfilamente - gedrückt durch benachbarte Verstärkungsdrähte - eher in die Höhe verteilen. Das Verhältnis ist mindestens 1,1, insbesondere 1,2, insbesondere 1,5, insbesondere 2. Die Obergrenze ist 3. Je nach Verbindungsart können sich die Filamenten aber auch weiterhin in Umfangsrichtung verteilen: Die Obergrenze des Verhältnisses ist 0,8, insbesondere 0,6, insbesondere 0,4. Die Untergrenze ist 0,2. Das Verhältnis kann auch 1 sein.

Als bevorzugtes Material der Einzelfilamente kommen bspw. in Frage:
Ultra High Molecular Weight Polyethylene (UHMWPE), Dyneema Purity®, biodegradierbare Polymere, Polyester, Polypropylen, Polyethylen, Polyurethan und Polytetrafluoroethylen. Der Verstärkungsdraht kann bspw. aus Nitinol, DFT (Außen NiTi-Legierung, Innen Platin oder Tantal), CoCr, Edelstahl gefertigt sein.

Folgende Geflechtdurchmesser für unterschiedliche Behandlungen werden im Zusammenhang mit der Erfindung und den hier offenbarten Ausführungsformen und Beispielen offenbart:
- Geflechtdurchmesser für Behandlung im intrakraniellen Bereich oder in der Halsschlagader: Obergrenze 6 mm, insbesondere 5,5 mm, insbesondere 5,0 mm. Untergrenze 3 mm.
- Geflechtdurchmesser für Behandlung von besonders kleinen intrakraniellen Gefäßen: Obergrenze 4,5 mm, insbesondere 4,0 mm, insbesondere 3,5 mm, insbesondere 3,0 mm. Untergrenze 2 mm.
- Geflechtdurchmesser für die okkludierende Behandlung innerhalb von Aneurysmen oder Hohlräumen: Obergrenze 3,0 mm, insbesondere 2,0 mm. Untergrenze 1,0 mm.
- Geflechtdurchmesser einer kugelförmigen Okklusionseinrichtung zum Verschluss innerhalb eines sehr großen, intrakraniellen Aneurysmas oder innerhalb des linken Vorhofohrs: Obergrenze 60 mm, insbesondere 50 mm, insbesondere 40 mm, insbesondere 30 mm, insbesondere 20 mm, insbesondere 15 mm. Untergrenze 10 mm.
- Geflechtdurchmesser einer kugelförmigen Okklusionseinrichtung zum Verschluss innerhalb eines großen bis kleinen, intrakraniellen Aneurysmas: Obergrenze 20 mm, insbesondere 18 mm, insbesondere 15 mm, insbesondere 12 mm, insbesondere 10 mm. Untergrenze 6 mm.
- Geflechtdurchmesser einer kugelförmigen Okklusionseinrichtung zum Verschluss innerhalb eines kleinen, intrakraniellen Aneurysmas: Obergrenze 12 mm, insbesondere 10 mm, insbesondere 0,8 mm, insbesondere 0,6 mm. Untergrenze 3 mm.
- Geflechtdurchmesser für Behandlung in der Aorta: Obergrenze 40 mm. Untergrenze 15 mm, insbesondere 20 mm, insbesondere 25 mm, insbesondere 30 mm.

Die Untergrenze der Anzahl der Verstärkungsdrähte 10 im Geflecht kann 6 Drähte, insbesondere 9 Drähte, insbesondere 12 Drähte, insbesondere 16 Drähte, insbesondere 24 Drähte betragen. Die Obergrenze kann bei 32 Drähten liegen.

Die Untergrenze der Anzahl der Faserstränge 11 kann 12 Faserstränge, insbesondere 18 Faserstränge, insbesondere 24 Faserstränge, insbesondere 32 Faserstränge, insbesondere 40 Faserstränge, insbesondere 48 Faserstränge, insbesondere 60 Faserstränge, insbesondere 72 Faserstränge, insbesondere 96 Faserstränge betragen. Die Obergrenze kann 128 Faserstränge betragen.

Die Untergrenze des Verhältnisses Verstärkungsdrahtanzahl/Faserstranganzahl beträgt 1:2, insbesondere 1:3, insbesondere 1:4, insbesondere 1:6, insbesondere 1:8. Die Obergrenze beträgt 1:12. Die maximale Anzahl der Faserstränge sollte besonders bevorzugt das Vierfache der Anzahl der Verstärkungsdrähte betragen. Bspw. können pro Verstärkungsdraht 10 zwei Faserstränge 11 geflochten und 2 Faserstränge 11 parallel mitlaufen, ohne geflochten zu sein.

Die Untergrenze des Flechtwinkels im expandierten Zustand beträgt 45°, insbesondere 50°, insbesondere 55°, insbesondere 60°, insbesondere 65°, insbesondere 70°, insbesondere 75°. Die Obergrenze beträgt 80°.

Die Untergrenze des Durchmessers der Verstärkungsdrähte beträgt 25 µm, insbesondere 30 µm, insbesondere 35 µm, insbesondere 40 µm, insbesondere 50 µm, insbesondere 60 µm, insbesondere 70 µm. Die Obergrenze beträgt 150 µm, insbesondere 120 µm, insbesondere 100 µm, insbesondere 80 µm.

Die Flechtart kann 1 über 2 sein. Die Verstärkungsdrähte sind dann beweglicher als bspw. bei einer 1 über 1 Verbindung. Die Einzelfilamente können sich besser verteilen und die Komprimierung im Katheter ist begünstigt.

Die Bindungsart ist bevorzugt axial, wodurch die Komprimierbarkeit in Katheter verbessert wird.

Vorzugsweise verlaufen mindestens 1, insbesondere mindestens 2 Faserstränge parallel zum 1 Verstärkungsdraht, die dem Verlauf des Drahtes folgen. Weitere Faserstränge können geflochten sein. Konkret verlaufen 2 Faserstränge parallel und flechtfrei zum Verstärkungsdraht. 2 weitere Faserstränge pro Verstärkungsdraht werden geflochten. Innerhalb einer virtuellen, aus Verstärkungsdrähten bestehenden Zelle befinden sich somit 4 Fasern.

Die Fasern und Drähte können an einem Ende offen sein und am anderen Ende Schlaufen bilden. Es ist auch möglich, dass die Fasern an beiden Seiten offen sind (Vereinfachung der Herstellung). Es ist ferner möglich, dass die Verstärkungsdrähte an beiden Seiten Schlaufen bilden, die Fasern aber nur ein einem oder an gar keinem Ende. Außerdem ist es möglich, dass die Fasern und Drähte an den Enden zumindest teilweise, vorzugsweise vollständig, Schlaufen bilden.

Bei der Herstellung eines erfindungsgemäßen Geflechtes ist es möglich, dass in einem ersten Schritt der Verstärkungsdraht/die Verstärkungsdrähte geflochten wird/werden und an den freien Enden, insbesondere mit Crimphülsen, verbunden werden. In einem anschließenden Herstellungsschritt werden die Faserstränge in die vom mindestens einen Verstärkungsdraht gebildeten Maschen bzw. Zellen eingeflochten und an den Enden in Schlaufen gelegt. Abhängig von der gewählten Flechtart, z. B. zwei über eins, werden die Faserstränge dann oben oder unten weitergeführt. Demnach kann ein Faserstrang durch jede Zelle verlaufen, wobei die Enden dann wieder zueinander geführt werden. Sofern mehrere Faserstränge durch eine Zelle bzw. durch eine Masche verlaufen, können die Faserstränge separat, d.h. unabhängig voneinander, in die vom Verstärkungsdraht gebildeten Maschen bzw. Zellen eingeflochten werden. Jeder Faserstrang kann durch jede Masche bzw. jede Zelle verlaufen.

Die Bildung von Schlaufen bei Verstärkungsdrähten minimiert das Risiko der Gefäßverletzung. Es ist auch möglich, dass mindestens ein Verstärkungsdraht keine Schlaufe bildet. Sofern ein Verstärkungsdraht keine Schlaufe bildet, sollte in diesem Bereich zumindest eine Drahtverbindung, insbesondere durch eine Hülse, ausgebildet sein. Es kann auch sein, dass die Enden der Fasern, als offene Enden oder auch als Schlaufen, sich innerhalb des Geflechtes befinden, das aus Verstärkungsdrähten besteht. Das bedeutet, dass die Fasern nur einen Bereich der Struktur abdecken, während die Enden des Geflechtes frei von Fasern sind. Dadurch können mittige Bereiche, z.B. im Bereich einer Stenose oder eines Aneurysma, abgedeckt werden, während die Enden die Durchblutung von Seitenästen und Perforatoren offen lassen. Das heißt nicht, dass im Bereich der Fasern keine Durchblutung in Seitenästen erfolgt. Nur herrscht hier ein etwas höherer Widerstand gegen den Blutfluss.

Der maximale Durchmesser des Innenlumens des Katheters beträgt 1 mm, insbesondere 0,7 mm, insbesondere 0,51 mm, insbesondere 0,42 mm. Die Untergrenze beträgt 0,35 mm.

## Patentansprüche

1. Medizinische Vorrichtung, insbesondere Stent, mit einem komprimierbaren und expandierbaren Geflecht, das wenigstens einen Verstärkungsdraht (10) und wenigstens einen Faserstrang (11) aufweist, wobei der Faserstrang (11) durch mehrere Einzelfilamente (12) gebildet ist,
**dadurch gekennzeichnet, dass**
die Einzelfilamente (12) im Faserstrang (11) derart lose angeordnet sind, dass die Einzelfilamente des Faserstrangs (11) im komprimierten Zustand des Geflechtes derart zusammengedrückt werden, dass diese aneinander anliegen und einen komprimierten Fasterstrang bilden und im expandierten Zustand des Geflechtes der Faserstrang verbreitert wird, wobei das Verhältnis von h/b_{expandiert} zu h/b komprimiert von 0,01 bis 0,8 beträgt, wobei h die Höhe des Faserstranges und b die Breite des Faserstranges bedeuten.

2. Vorrichtung nach Anspruch 1,
**dadurch gekennzeichnet, dass**
das Geflecht wenigstens einen Verstärkungsdraht (10), insbesondere wenigstens zwei Verstärkungsdrähte (10) aufweist, der bzw. die zur Bildung von Maschen geflochten ist bzw. sind.

3. Vorrichtung nach Anspruch 2,
**dadurch gekennzeichnet, dass**
die Einzelfilamente (12) des Faserstranges (11) oder die Einzelfilamente (12) mehrerer Faserstränge (11) in wenigstens einer Masche im expandierten Zustand des Geflechtes zumindest teilweise verteilt, insbesondere homogen verteilt, angeordnet sind.

4. Vorrichtung nach einem der vorhergehenden Ansprüche,
**dadurch gekennzeichnet, dass**
der Faserstrang (11) im expandierten Zustand breiter als im komprimierten Zustand ist und/oder die Einzelfilamente zumindest teilweise planar angeordnet sind.

5. Vorrichtung nach einem der vorhergehenden Ansprüche,
**dadurch gekennzeichnet, dass**
der Faserstrang (11) von 10 bis 50 Einzelfilamente (12), insbesondere von 20 bis 30 Einzelfilamente (12) aufweist.

6. Vorrichtung nach einem der vorhergehenden Ansprüche,
**dadurch gekennzeichnet, dass**
das Einzelfilament (12) einen runden, insbesondere kreisrunden, Querschnitt mit einem Durchmesser von 5 bis 30 µm, insbesondere von 10 bis 20 µm aufweist.

7. Vorrichtung nach einem der vorhergehenden Ansprüche,
**dadurch gekennzeichnet, dass**
der Verdrillungsgrad in Umdrehungen pro Meter eines Faserstranges (11) 50 bis 800, insbesondere 50 bis 700, insbesondere 50 bis 600, insbesondere 50 bis 500 beträgt.

8. Vorrichtung nach einem der vorhergehenden Ansprüche,
**dadurch gekennzeichnet, dass**
die Anzahl der Verstärkungsdrähte (10) von 6 bis 32 beträgt und/oder die Anzahl der Faserstränge (11) von 12 bis 128 beträgt.

9. Vorrichtung nach einem der vorhergehenden Ansprüche,
**dadurch gekennzeichnet, dass**
das Verhältnis der Anzahl von Verstärkungsdraht (10) zu Faserstrang (11) von 1:12 bis 1:2 beträgt.

10. Vorrichtung nach einem der vorhergehenden Ansprüche,
**dadurch gekennzeichnet, dass**
der Flechtwinkel im expandierten Zustand von 45° bis 80° beträgt.

11. Vorrichtung nach einem der vorhergehenden Ansprüche,
**dadurch gekennzeichnet, dass**
der Durchmesser des Verstärkungsdrahtes (10) von 25 µm bis 150 µm, insbesondere von 35 µm bis 100 µm beträgt.

12. Vorrichtung nach einem der vorhergehenden Ansprüche,
**dadurch gekennzeichnet, dass**
der wenigstens eine Verstärkungsdraht (10) und der wenigstens eine Faserstrang (11) in der Flechtart 1 über 2 angeordnet sind.

13. Vorrichtung nach einem der vorhergehenden Ansprüche,
**dadurch gekennzeichnet, dass**
wenigstens ein Faserstrang (11) parallel zum dem wenigstens einen Verstärkungsdraht (10) und/oder wenigstens ein weiterer Faserstrang (11) zumindest mit dem Verstärkungsdraht (10) verflochten ist.

14. Vorrichtung nach einem der vorhergehenden Ansprüche,
**dadurch gekennzeichnet, dass**
wenigstens ein Faserstrang (11) unterschiedliche Einzelfilamente (12), insbesondere Einzelfilamente (12) mit wenigstens einem unterschiedlichen Durchmesser, aufweist und/oder mehrere Faserstränge (11) unterschiedlich viele Einzelfilamente (12) aufweisen.

15. Vorrichtung nach einem der vorhergehenden Ansprüche,
**dadurch gekennzeichnet, dass**
wenigstens ein Verstärkungsdraht (10) an wenigstens einem Ende des Geflechtes eine Schlaufe oder verbundene Drahtenden aufweist.

16. Vorrichtung nach einem der vorhergehenden Ansprüche,
**dadurch gekennzeichnet, dass**
wenigstens ein Faserstrang (11) an wenigstens einem Ende des Geflechtes eine Schlaufe oder verbundene Einzelfilamentenden aufweist.

17. System aufweisend eine medizinische Vorrichtung, insbesondere Stent, nach einem der vorhergehenden Ansprüche und ein Zufuhrsystem, insbesondere eine Schleuse und einen Führungsdraht, wobei der Außendurchmesser der Vorrichtung, insbesondere des Stents, im expandierten Ruhezustand höchstens 6 mm und der Innendurchmesser des Zufuhrsystems höchstens 0,7 mm betragen.

## Claims

1. Medical device, in particular stent, with a compressible and expandable mesh which comprises at least one reinforcing wire (10) and at least one fibre bundle (11), wherein the fibre bundle (11) is formed of several individual filaments (12)
**characterised in that**
the individual filaments (12) in the fibre bundle (11) are arranged so loosely that in the compressed state of the mesh the individual filaments of the fibre bundle are pressed together in such a way that they are in contact with each other and form a compressed fibre bundle and in the expanded state of the mesh the fibre bundle broadens out, wherein the ratio of h/b_{expanded} to h/b_{compressed} is from 0.01 to 0.8, were h denotes the height of the fibre bundle and b the breadth of the fibre bundle.

2. Device according to claim 1
**characterised in that**
the mesh comprises at least one reinforcing wire (10), more particularly two reinforcing wires (10), which is/are braided to produce mesh loops.

3. Device according to claim 2
**characterised in that**
in the expanded state of the mesh the individual filaments (12) of the fibre bundle (10) or the individual filaments (12) of several fibre bundles (11) in at least one mesh loop are arranged in an at last partially distributed, in particular homogeneously distributed manner.

4. Device according to any one of the preceding claims **characterised in that**
in the expanded state the fibre bundle (11) is broader than in the compressed state and/or the individual filaments are at least partially arranged in a planar manner.

5. Device according to any one of the preceding claims **characterised in that**
the fibre bundle (11) comprises from 10 to 50 individual filaments (12), more particularly 20 to 30 individual filaments (12).

6. Device according to any one of the preceding claims **characterised in that**
the individual filament (12) has a round, more particularly circular, cross-section with a diameter from 5 to 30 µm, more especially from 10 to 20 µm.

7. Device according to any one of the preceding claims **characterised in that**
the twisting rate in turns per metre of a fibre bundle (11) is 50 to 800, in particular 50 to 700, in particular 50 to 600, in particular 50 to 500.

8. Device according to any one of the preceding claims **characterised in that**
the number of reinforcing wires (10) is from 6 to 32 and/or the number of fibre bundles (11) is from 12 to 128.

9. Device according to any one of the preceding claims **characterised in that**
the ratio of the number of reinforcing wires (10) to fibre bundles (11) is 1:12 to 1:2.

10. Device according to any one of the preceding claims **characterised in that**
the braiding angle in the expanded state is from 45° to 80°.

11. Device according to any one of the preceding claims **characterised in that**
the diameter of the reinforcing wire (10) is from 25 µm to 150 µm, in particular from 35 µm to 100 µm.

12. Device according to any one of the preceding claims **characterised in that**
the at least one reinforcing wire (10) and the at least one fibre bundle (11) are arranged in the braiding manner 1 over 2.

13. Device according to any one of the preceding claims **characterised in that**
at least one fibre bundle (11) is braided in parallel to the at least one reinforcing wire (10) and/or at least one further fibre bundle (11) is braided at least with the reinforcing wire (10).

14. Device according to any one of the preceding claims **characterised in that**
at least one fibre bundle (11) comprises various individual filaments (12), in particular individual filaments (12) with at least one different diameter and/or several fibre bundles (11) have different quantities of individual filaments (12).

15. Device according to any one of the preceding claims **characterised in that**
at least one reinforcing wire (10) has a loop or connected wire ends at at least one end of the mesh.

16. Device according to any one of the preceding claims **characterised in that**
at least one fibre bundle (11) has a loop or connected individual filament ends at at least one end of the mesh.

17. System comprising a medical device, in particular a stent, according to any one of the preceding claims and an introduction system, in particular a lock and a guide wire, wherein the outer diameter of the device, in particular the stent, in the expanded resting state is at most 6 mm and the inner diameter of the introduction system is at most 0.7 mm.

## Revendications

1. Dispositif médical, notamment endoprothèse vasulaire, avec un tressage comprimable et expansible, qui comporte au moins un fil métallique de renfort (10) et au moins un faisceau de fibres (11), le faisceau de fibres (11) étant formé par plusieurs filaments individuels (12),
**caractérisé en ce que** les filaments individuels (12) sont placés en étant lâches dans le faisceau de fibres (11), de telle sorte que lorsque que le tissage est à l'état comprimé, les filaments individuels du faisceau de fibre (11) soient comprimés de sorte à être mutuellement adjacents et forment un faisceau de fibres comprimé et lorsque le tissage est à l'état expansé, le faisceau de fibre soit élargi, le rapport de h/b_{expansé} à h/b_{comprimé} s'élevant à de 0,01 à 0,8, h étant la hauteur du faisceau de fibres et b étant la largeur du faisceau de fibres.

2. Dispositif selon la revendication 1,
**caractérisé en ce que** le tissage comporte au moins un fil métallique de renfort (10), notamment au moins deux fils métalliques de renfort (10) qui est ou qui sont tressé(s) pour former des mailles.

3. Dispositif selon la revendication 2,
**caractérisé en ce que** dans au moins une maille, lorsque le tissage est à l'état expansé, les filaments individuels (12) du faisceau de fibres (11) ou les filaments individuels (12) de plusieurs faisceaux de fibres (11) sont placés en étant au moins en partie distribués, notamment distribués de manière homogène.

4. Dispositif selon l'une quelconque des revendications précédentes,
**caractérisé en ce que** lorsqu'il est à l'état expansé, le faisceau de fibres (11) est plus large qu'à l'état comprimé et/ou **en ce que** les filaments individuels sont placés au moins en partie de manière planaire.

5. Dispositif selon l'une quelconque des revendications précédentes,
**caractérisé en ce que** le faisceau de fibres (11) comporte de 10 à 50 filaments individuels (12), notamment de 20 à 30 filaments individuels (12).

6. Dispositif selon l'une quelconque des revendications précédentes,
**caractérisé en ce que** le filament individuel (12) présente une section transversale ronde, notamment circulaire, avec un diamètre de 5 à 30 µm, notamment de 10 à 20 µm.

7. Dispositif selon l'une quelconque des revendications précédentes,
**caractérisé en ce que** le pas de torsion en tours au mètre d'un faisceau de fibres (11) s'élève à de 50 à 800, notamment de 50 à 700, notamment de 50 à 600, notamment de 50 à 500.

8. Dispositif selon l'une quelconque des revendications précédentes,
**caractérisé en ce que** le nombre des fils métalliques de renfort (10) s'élève à de 6 à 32 et/ou le nombre des faisceaux de fibres (11) s'élève à de 12 à 128.

9. Dispositif selon l'une quelconque des revendications précédentes,
**caractérisé en ce que** le rapport du nombre du fil métallique de renfort (10) au faisceau de fibres s'élève à de 1 : 12 à 1 : 2.

10. Dispositif selon l'une quelconque des revendications précédentes,
**caractérisé en ce que** l'angle de tressage dans l'état expansé s'élève à de 45° à 80°.

11. Dispositif selon l'une quelconque des revendications précédentes,
**caractérisé en ce que** le diamètre du fil métallique de renfort 10) s'élève à de 25 µm à 150 µm, notamment de 35 µm à 100 µm.

12. Dispositif selon l'une quelconque des revendications précédentes,
**caractérisé en ce que** l'au moins un fil métallique de renfort (10) et l'au moins un faisceau de fibres (11) sont placés selon le mode de tressage 1 sur 2.

13. Dispositif selon l'une quelconque des revendications précédentes,
**caractérisé en ce qu'**au moins un faisceau de fibres (11) est tressé à la parallèle de l'au moins un fil métallique de renfort (10) et/ou **en ce qu'**au moins un faisceau de fibres (11) supplémentaire est tressé au moins avec le fil métallique de renfort (10).

14. Dispositif selon l'une quelconque des revendications précédentes,
**caractérisé en ce qu'**au moins un faisceau de fibres (11) comporte différents filaments individuels (12), notamment des filaments individuels (12) avec au moins un diamètre différent et/ou **en ce que** plusieurs faisceaux de fibres (11) comportent un nombre différent de filaments individuels (12).

15. Dispositif selon l'une quelconque des revendications précédentes,
**caractérisé en ce que** sur au moins une extrémité du tressage, au moins un fil métallique de renfort (10) comporte une boucle ou des extrémités de fil métallique reliées.

16. Dispositif selon l'une quelconque des revendications précédentes,
**caractérisé en ce que** sur au moins une extrémité du tressage, au moins un faisceau de fibres (11) comporte une boucle ou des extrémités de faisceau de fibres reliées.

17. Système comportant un dispositif médical, notamment une endoprothèse vasculaire selon l'une quelconque des revendications précédentes et un système d'amenage, notamment un sas de transfert et un fil métallique de guidage, le diamètre extérieur du dispositif, notamment de l'endoprothèse vasculaire s'élevant à un maximum de 6 mm et le diamètre intérieur du système d'amenage s'élevant à un maximum de 0,7 mm.
